# EUROPEAN PATENT SPECIFICATION

(11) **EP 2 618 832 B1**
(45) Date of publication and mention of the grant of the patent: **07.06.2023**
(21) Application number: 11761575.7
(22) Date of filing: 22.09.2011
(51) Int. Cl.: A61K 38/44, A61K 31/07, A61P 27/02

(54) **COMPOSITION COMPRISING SOD, LUTEIN AND ZEAXANTHIN**
ZUSAMMENSETZUNG MIT SOD, LUTEIN UND ZEAXANTHIN
COMPOSITION COMPRENANT DE LA SOD, DE LA LUTÉINE ET DE ZÉAXANTHINE

(30) Priority: 24.09.2010 EP 10306032
(43) Date of publication of application: 31.07.2013
(73) Proprietor: OmniVision GmbH, 82178 Puchheim (DE)
(72) Inventor: LEPELLETIER, Yves, F-91200 Athis-Mons (FR); HADJ-SLIMANE, Reda, F-75015 Paris (FR); HADJ-SLIMANE, Tewfik, 48000 Relizane (DZ)
(74) Representative: Blodig, Wolfgang
(86) International application number: PCT/EP2011/066539
(87) International publication number: WO 2012/038512

(56) References cited:
- WO-A1-96/21462
- WO-A1-02/098345
- WO-A2-03/082081
- US-A1- 2005 032 768
- US-A1- 2007 265 351
- US-A1- 2010 159 029
- LEE SUNGMUN ET AL: "Polyketal microparticles: A new delivery vehicle for superoxide dismutase", BIOCONJUGATE CHEMISTRY, vol. 18, no. 1, January 2007 (2007-01), pages 4-7, XP002630444, ISSN: 1043-1802
- VOULDOUKIS IOANNIS ET AL: "Antioxidant and anti-inflammatory properties of a Cucumis melo LC. extract rich in superoxide dismutase activity", JOURNAL OF ETHNOPHARMACOLOGY, ELSEVIER SCIENTIFIC PUBLISHERS LTD, IE, vol. 94, no. 1, 1 September 2004 (2004-09-01), pages 67-75, XP002321961, ISSN: 0378-8741, DOI: 10.1016/J.JEP.2004.04.023
- STEPHEN BEATTY ET AL: "The Role of Oxidative Stress in the Pathogenesis of Age-Related Macular Degeneration", SURVEY OF OPHTHALMOLOGY, vol. 45, no. 2, 1 September 2000 (2000-09-01), pages 115-134, XP55020899, ISSN: 0039-6257, DOI: 10.1016/S0039-6257(00)00140-5
- DATABASE GNPD [Online] MINTEL; 15 July 2009 (2009-07-15), anonymous: "Eye Sight Improving Dietary Supplement", XP055656250, retrieved from www.gnpd.com Database accession no. 1126829
- DATABASE GNPD [Online] MINTEL; 22 June 2010 (2010-06-22), anonymous: "Intestinal Function Dietary Supplement", XP055656306, retrieved from www.gnpd.com Database accession no. 1345985

## Description

### FIELD OF INVENTION

The present invention relates to a composition comprising the enzyme superoxide dismutase (SOD) in association with lutein and at least one stereoisomer of zeaxanthin. Said composition is a nutraceutical composition or a pharmaceutical composition, and promotes or maintains visual health particularly in AMD, through the inhibition of oxidative damages. The invention also relates to a kit of parts comprising the composition. The invention further relates to the use of a functional food, nutraceutical composition, or dietary supplement comprising the composition, as well as to a pharmaceutical composition comprising the composition. Moreover, the invention relates to a medicament comprising the kit of parts or the composition.

### BACKGROUND OF THE INVENTION

In developed countries, the most frequent causes of visual incapacities or blindness are diseases, such as for example age-related macular degeneration (AMD), diabetic retinopathies (DR), cataract and retinitis pigmentosa (RP). These different ocular pathologies present the common characteristic to be associated with oxidative stress.

Oxidative stress corresponds to an excess of oxidant elements as compared to antioxidant products, resulting in structural or formal damages, such as cellular damages. Oxidative stress is linked to an excessive production of reactive oxygen species (ROS) coupled to a deficiency in anti-radical defenses. In particular, oxidative stress leads to the accumulation of toxic superoxide ion (O₂⁻). In retina, the oxidation of lipids linked to amino acids leads to the accumulation of lipofuscine, a fluorescent pigment. This pigment induces the production of ROS leading to cell death by apoptosis (Wihlmark et al., Free radic. Biol. Med. 1997; 22: 1229-34). Moreover, with ageing, antioxidant defenses decrease, in particular in the eye.

With global ageing of the population, the occurrence of the cited diseases increases. The provoked symptoms are highly invalidating for patients. There is, thus, a real need to develop strategies to counterbalance oxidative stress in the eye, in order to prevent and/or treat said diseases.

In human, antioxidant protection involves two major types of elements: enzymes or non-enzymatic products.

Three major antioxidant enzymes are present in human cells: glutathione reductase and peroxydase (GPX), superoxide dismutase (SOD) and catalase (CAT). The combination of the action of these three enzymes leads to the transformation of highly toxic superoxide ion to hydrogen peroxide (H₂O₂) and finally to water, as shown by the chemical reactions below.

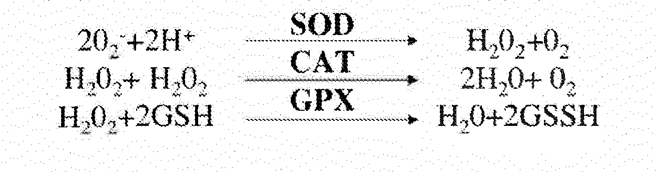

The use of SOD, for example from *Cucumis melo* LC., to treat diseases associated with oxidative stress is well known in the prior art. US2003/0228299 describes the use of pharmaceutical compositions comprising SOD for topical administration, in order to prevent and/or treat superficial eye disorders. Moreover, WO03/082081 describes the intravitreal, topical or systemic use of a compound (such as, for example, SOD) effective to reduce the amount of Reactive Oxygen Metabolite for treating intraocular damages in a subject in need thereof. However, oral administration of SOD has longer been limited, due to the degradation of the enzyme through the digestive tract (Zidenberg-Cherr et al., The American Journal of Clinical Nutrition, 1983). US 6,045,809 and Dugas (Free Radic Biol Med, 2002), both describe the use of gliadin biopolymers to protect SOD from digestive degradation. A form of SOD protected by gliadin is available as GliSODin^{®} (Isocell). The catalytic activity of SOD protected by gliadin is preserved during oral administration (Vouldoukis et al., Journal of Ethnopharmacology, 2004), allowing its administration through an oral way.

In parallel to enzymatic protection of cells against oxidative stress, some non-enzymatic products exist. For example, vitamins (A, E or C), zinc, selenium, anthocyanins or macular pigments, such as, for example, lutein and zeaxanthin are usually used for their antioxidant properties. Lutein and zeaxanthin are macular pigments representing 99% of total pigments of the macula, which belong to the family of carotenoids, and specifically to the family of xanthophylls. Both pigments may exhibit on one hand an indirect antioxidant effect through their capacity to absorb blue light particularly aggressive for photoreceptor and on the other hand direct antioxidant properties. US2005/0032914, US2010/0159029 and WO2009/129859 describe compositions for oral administration, comprising lutein and zeaxanthin in combination with other non-enzymatic antioxidant products, for enhancing visual performance, inhibiting macular degeneration or promoting eye health. US2007/265351 describes a xanthophyll composition comprising lutein and zeaxanthin useful for eye health. Moreover, lutein is defined by the Afssa (Agence Française de Sécurité Sanitaire des Aliments) as "an agent which contributes to protect retina and lens against oxidation" (Saisine n°2003-SA-0205, 2004) and scientific studies have shown a relationship between the dietary intake of lutein/zeaxanthin and the likelihood of having AMD (SanGiovanni et al., AREDS report n°22, Arch Ophthalmol, 2007).

CN 101243875 describes a health preparation, comprising lutein and beta-carotene to enhance the activity of SOD. The Applicant discloses the fact that lutein alone is less efficient to enhance the activity of SOD than in combination with betacaroten.

Surprisingly, and contrary to the teaching of the prior art, especially CN 101243875, the Applicant identified an unexpected synergic effect of oral administration of SOD protected by gliadin, lutein and at least one isomer of zeaxanthin on global health, and on the protection against oxidative stress, without the addition of beta-carotene. Moreover, the Applicant carried out experiments showing that this synergy is enhanced when components are administered to a subject non-simultaneously.

### SUMMARY

This invention thus relates to a composition comprising an enzyme selected from the group comprising superoxide dismutase (SOD) in association with lutein and at least one stereoisomer of zeaxanthin for the uses defined in claims 1, 2, and 24. The invention also provides the kit of parts of claim 11, the use of claim 14, the pharmaceutical compositions of claims 17 and 18, and the medicaments of claims 19 and 20.

Advantageously, the enzyme is of natural origin, and the composition comprises a vegetal extract including SOD, preferably an extract of *Cucumis melo* LC, preferably the amount of vegetal extract including superoxide dismutase ranges from 10 to 2000 mg, preferably from 50 to 1000 mg, more preferably 100-500 mg. According to a preferred embodiment, the enzymatic activity of said enzyme is of at least 1.1 UI/mg. Advantageously, the enzyme is micro-encapsulated with a biopolymer, preferably a prolamine or PCADK, and optionally the micro-encapsulated enzyme is further coated with a polymer, preferably chitosan or hyaluronic acid.

In an embodiment, the at least one zeaxanthin isomer is selected from the group consisting of zeaxanthin and mesozeaxanthine. In a further embodiment, the amount of lutein ranges from 0.5 to 25 mg, and the amount of at least one zeaxanthin isomer ranges from 0.1-5 mg. Advantageously, lutein and at least one zeaxanthin isomer are present in the composition in a ratio lutein:zeaxanthin isomer of about 5: 1.

In a preferred embodiment, the association is an intimate mixture of superoxide dismutase, lutein and at least one zeaxanthin stereoisomer.

This invention also relates to a kit of parts comprising a composition as described here above, wherein the kit comprises a first part comprising the enzyme superoxide dismutase, and a second part comprising lutein and at least one zeaxanthin stereoisomer. Advantageously, one of the first and second parts is formulated for sustained release thereof. In an embodiment, the first and second parts are administered to an individual simultaneously or sequentially, and when administered sequentially, the first part comprising the enzyme is administered 12 hours before or after the second part comprising lutein and at least one zeaxanthin isomer.

This invention also relates to a use of a functional food, a nutraceutical composition or a food or dietary supplement comprising a composition or a kit of parts as described here above. Advantageously, the functional food, the nutraceutical composition or the food or dietary supplement comprises 50-200 mg, preferably about 100 mg enzyme, 2.5-7.5 mg, preferably about 5 mg lutein and 0.5-1.5 mg, preferably about 1 mg zeaxanthin. It may be in a form suitable for being orally administered. In one embodiment, the functional food, the nutraceutical composition or the food or dietary supplement is for maintaining or improving visual comfort in a subject.

This invention also relates to a pharmaceutical composition comprising a composition or a kit of parts as described here above in association with at least one pharmaceutically acceptable excipient. This invention also relates to a medicament comprising a composition or a kit of parts as described here above. The pharmaceutical composition or the medicament according to the invention may comprise 100-1000 mg superoxide dismutase, 1-25 mg lutein and 0.5-5 mg zeaxanthin. They may be in a form suitable for being orally, topically, intraocularly or systemically administered.

Also described is a method for, or a composition for use in or for maintaining visual health and/or for use in treating, preventing or stabilizing a disease, condition or disorder of the eye associated to oxidative stress, comprising administering to a subject in need thereof a pharmaceutical composition or a medicament as described here above. The subject may be affected by a disease associated with oxidative stress, especially Age-related Macular Degeneration (AMD), diabetic retinopathies, cataract or retinitis pigmentosa.

According to an embodiment, the subject is an animal, preferably a mammal, such as, for example, cat, dog, hamster, rabbit, mouse, gerbil, rat, Guinea pig, human, more preferably the subject is a human.

### DEFINITIONS

In the present invention, the following terms have the following meanings:
- **"Antioxidant"** refers to an enzymatic or non-enzymatic agent that diminishes or avoids the oxidation of other substances. An antioxidant may protect cells against the effect of reactive oxygen species.
- **"Oxidative stress"**: imbalance between the production of reactive oxygen species on one hand and on the other hand the detoxification of the reactive intermediates and/or the repair of the resulting damages.
- **"Oral administration"**: the administration of a product in the mouth cavity followed by the swallowing of the product. The substance reaches the systemic circulation through a digestive absorption.
- **"Topical administration"**: defines the delivery of a product by application to the skin or mucous membrane, preferably a topical administration refers to the application of a product on the surface of an eye.
- **"Intraocular administration"**: refers to the injection of a product in the interior of the eye, wherein the interior of the eye means any area located within the eyeball, and which generally includes, but is not limited to, any functional (e.g. for vision) or structural tissues found within the eyeball, or tissues or cellular layers that partially or completely line the interior of the eyeball. Specific examples of such areas include the anterior chamber, the posterior chamber, the vitreous cavity, the choroid, the macula, and the retina, and blood vessels and nerves, which vascularize or innervate a posterior ocular region or site. According to a preferred embodiment, interior of the eye means the posterior segment of the eye, including the posterior chamber, the vitreous cavity, the choroid, the macula, and the retina, and blood vessels and nerves, which vascularize or innervate a posterior ocular region or site.

According to this preferred embodiment, the intraocular administration refers to an administration within the posterior segment of the eye, preferably within the vitreous, and the intraocular administration is more preferably an intravitreal injection.
- **"Systemic injection"**: refers to the injection of a product in the animal body, wherein the product reaches the systemic circulation.
- **"Functional food"**: any modified food or food ingredient that may provide a benefit or provide protection against physiological disorder or discomfort; beyond the traditional nutrients it contains.
- **"Nutraceutical product"**: a product isolated or purified from comestibles. A nutraceutical is demonstrated to have a physiological benefit or provide protection against physiological disorder or discomfort.
- **"Food or dietary supplement"**: a product that contains a vitamin, mineral, herb or other botanical, amino acid, concentrate, metabolite, constituent, extract, or combinations of these ingredients.
- **"Polymer":** large molecule formed by the repetition of a structural unit.
- **"Biopolymer":** defines a polymer produced by living organism, such as, for example, DNA, cellulose, starch, gliadin, chitosan, hyaluronic acid, cyclodextrine, polysome, etc...
- **"Sustained release":** describes the slow release kinetic of a compound, over an extended period of time, preferably 6 hours or more, more preferably 10 hours or more, even more preferably 12 hours or more.
- **"Synergy"**: defines the interaction of two or more agents acting together in a positive way to produce an effect that neither could produce alone. An **"additive synergy"** defines a synergy wherein the combined effect of the agents is equal to the sum of the effects of each agent alone. When the combined effect is greater than the sum of the effects of each agent operating by itself, the synergy is referred as a **"potentiating effect"**. In one embodiment, the synergy is an additive synergy. In another embodiment, the synergy is a potentiating effect.
- **"About"** preceding a figure means plus or less 10% of the value of said figure.
- **"Association":** refers to a combination of products, or to at least two products, which are to be administered or ingested by a subject in a simultaneous or sequential manner, in order to have a combined effect.
- **"Intimate mixture":** refers to the close union or combination of elements.
- **"UI":** 1 UI corresponds to the transformation of one micromole of substrate (ROS) per minute at 25°C.
- **"Visual discomfort":** refers to the absence or to a decrease in the feeling of ease or well-being. A visual discomfort may result from oxidative stress. An example of oxidative stress resulting in visual discomfort is the UV exposure inducing phototoxicity. A visual discomfort may be pathological or non-pathological.
- **"Pathological discomfort"**: defines a discomfort, which is a symptom of an ocular disease, disorder or condition. Examples of pathological visual discomforts include, but are not limited to, pain, symptomatic loss of visual acuity such as, for example, blurred or decreased central close-up and distance vision observed in AMD; floaters observed in diabetic retinopathies and the like.
- **"Non-pathological discomfort":** defines a discomfort which is not related to an ocular disease, disorder or condition. In one embodiment, the term "non-pathological discomfort" may correspond to a loss of visual function or to the occurrence of a visual defect. As used herein, the expression "a loss of visual function" or "an occurrence of a visual defect" may encompass one or more of the following features: loss of visual acuity, decrease in the field of vision, loss of twilight vision, presence of glare and of contrast sensitivity and diplopia. Methods for measuring visual function are known from the skilled artisan. For example, visual acuity may be measured by ETDRS acuity testing (ETDRS stands for Early Treatment Diabetic Retinopathy Study); field of vision may be measured by perimetry or by the use of a campimeter; glare may be measured by having the subject adjust brightness of a light source until it reaches an ill-defined threshold of unpleasantness...
- **"Maintaining Visual health"** means preventing, stabilizing and/or treating ocular diseases, disorders or conditions, and/or alleviating or preventing a pathological visual discomfort related thereto.
   Examples of diseases, disorders or conditions include, but are not limited to, diseases, disorders or conditions related to oxidative stress, such as, for example, age related macular degeneration (AMD), diabetic retinopathy, cataract and retinitis pigmentosa.
   Maintaining visual health is a therapeutic action and may include treating a symptom of a disease, wherein the term "treating" also encompass a preventive action.
- **"Maintaining or improving visual comfort":** means alleviating a non-pathological visual discomfort or preventing its occurrence only, and excludes any therapeutic action. In one embodiment, the term "discomfort" may correspond to eye fatigue, which may easily be measured. Eye fatigue may include, but is not limited to, eye itching, eye pain, eye winking, enhanced or lowered tear production in the eye of a subject and the like.
   Maintaining or improving visual comfort is a non-therapeutic action and may include correcting a visual defect or a loss of visual function.
- **"Subject"** or **"individual"**: refers to an animal, preferably a mammal, more preferably a human, receiving the composition of the invention.
- **"SOD Mimics":** refers to molecules having a similar effect on protection from oxidative damage as SOD.

### DETAILED DESCRIPTION

The invention relates to a composition comprising the enzyme superoxide dismutase (SOD) in association with lutein and at least one stereoisomer of zeaxanthin.

In a first embodiment of the invention, SOD is synthetic SOD. Advantageously, synthetic SOD is recombinant SOD, provided from vegetal or animal, such as, for example human, sequences cloned and produced in a prokaryote system, such as, for example, a bacterial system; in an eukaryote system, such as, for example in a fungus, such as, for example, a yeast, or in mammalian cell such as, for example, CHO cells; or in a viral system, such as, for example, bacculovirus.

In a second embodiment, SOD is of natural origin. Advantageously, the composition comprises a natural extract including SOD, preferably the natural extract is a vegetal extract. In an embodiment, the vegetal extract comprising SOD is not from a genetically modified organism (GMO) or genetically engineered organism (GEO). According to the invention, said vegetal extract is selected from the group comprising Red Seaweed, Green Seaweed, Watermelon (*Citrullus vulgaris* Schrad) cotyledons, Oats, Peas, Corn and Melon. In a preferred embodiment, said vegetal extract is from melon, preferably from *Cucumis melo* LC. (Cucurbitaceae).

In a first embodiment of the invention, the amount of vegetal extract including SOD in the composition ranges from 10 to 500 mg, preferably from 50 to 200 mg, more preferably is about 100 mg. In this embodiment, preferably the percentage of vegetal extract including SOD ranges from 50-95% in weight to the weight of all active agents present in the composition.

In a second embodiment of the invention, the amount of vegetal extract including SOD in the composition ranges from 50 to 2000 mg, preferably from 100 to 1000 mg, more preferably of about 250 mg. In this embodiment, preferably the percentage of vegetal extract including SOD ranges from 65-95% in weight to the weight of all active agents present in the composition. Preferably, the percentage of vegetal extract including SOD ranges from 30-50% in weight to the total weight of the composition.
In a preferred embodiment, the enzymatic activity of the enzyme is of at least 0.5 UI/mg, preferably of at least 1 UI/mg, more preferably of at least 1.1 UI/mg.

Superoxide dismutase is a metalloprotein, which exists in several forms, dependant on the metal present in the molecule (Zn, Cu, Fe, Mn or Ni). The two principal forms of SOD are Cu/Zn SOD and Mn SOD. The first one, Cu/Zn SOD, comprises two Cu²⁺ atoms and two Zn²⁺ atoms per molecule of enzyme, whereas the second one, Mn SOD, comprises Mn²⁺ atoms. Cu/Zn SOD shows the highest efficiency to counteract oxidation.

In an embodiment of the invention, SOD is Cu/Zn SOD and/or Mn SOD. In a preferred embodiment, SOD is Cu/Zn SOD.

The enzyme in the composition of the invention is a protein, and is thus degraded after oral ingestion, in the stomach and during digestion. In order to protect the enzyme against this degradation, the enzyme of the present invention may be protected by micro-encapsulation. Injection or ingestion of micro-encapsulated elements shall lead to the sustained release of the enzyme at the desired site, preventing any prior degradation of said element by the digestive apparel. Micro-encapsulation may be based on polymerization or reticulation.

According to the invention, the enzyme is micro-encapsulated.

In a first embodiment, the enzyme is micro-encapsulated in a biopolymer, preferably made of prolamin. The presence of prolamin may retain the active ingredient and delay its release in the small intestine. Advantageously, prolamin is extracted from a vegetal selected from the group comprising, but not limited to, rye (secalin), wheat (gliadin), oats (avenin), barley (hordein), barley herb, rice, birdseed and corn. In a preferred embodiment, said prolamin is gliadin, preferably extracted from wheat, more preferably wheat is selected from the group comprising *Triticum vulgare, Triticum durum, Triticum herbe, Triticum spelta* and *Triticum polonicum,* even more preferably wheat is *Triticum vulgare.* According to an embodiment, gliadin from *Triticum vulgare* is used in its native form, i.e. non denaturized. Wheat gliadin is commercially available. In an embodiment, wheat gliadin is Gliamine^{®} from HITEX (Vannes, France). In a preferred embodiment, the enzyme is SOD, and SOD micro-encapsulated by gliadin is GliSODin^{®}, provided by Isocell (Paris, France). GliSODin^{®} is a mix of Extramel^{®} (from Bionov company) with gliadin and maltodextrine. Extramel^{®} is an extract from *Cucumis melo* LC., that comprises 3 to 7-fold more SOD than other melon species. In an embodiment, prolamin may be provided in the form of a prolamin based peptide fragment, preferably a gliadin based peptide fragment. A gliadin based peptide fragment is a fragment of gliadin or a derivative, analog, salt or metabolite thereof. More preferably, prolamin based peptide fragment is a non-immunogenic analog of gliadin. In one embodiment, the prolamin based peptide fragment is chosen from the group consisting of fully hydrolyzed, substantially hydrolyzed or slightly hydrolyzed prolamin based peptide fragments.

In a second embodiment, the enzyme is micro-encapsulated with a biopolymer made of poly(cyclohexane-1,4-diyl acetone dimethyl ketal) (PCADK).

According to an embodiment, the micro-encapsulated enzyme is further coated with a polymer, which facilitates its digestive absorption and/or enhances its protection from degradation. In an embodiment, said polymer is selected from the group comprising povidone, chitosan, hyaluronic acid, polysome, phytoliposome, dextran, titanium dioxide, polysaccharide, lipids, phosphatidylcholine. In a preferred embodiment, said polymer is chitosan and/or hyaluronic acid.

In an embodiment, said particles of the micro-encapsulated enzyme have a size ranging from 200 nM to 1000 µM.

In an embodiment of the invention, the at least one zeaxanthin isomer is selected from the group consisting of (3R, 3'R) zeaxanthin, (3R, 3'S) zeaxanthin (mesozeaxanthine) and (3S, 3'S) zeaxanthine isomer; preferably, the zeaxanthin isomer is zeaxanthin and/or mesozeaxanthin. Mesozeaxanthin is a stereoisomer of zeaxanthin, synthesized in the eye from lutein.

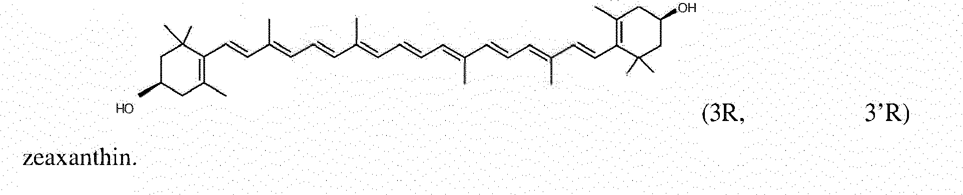

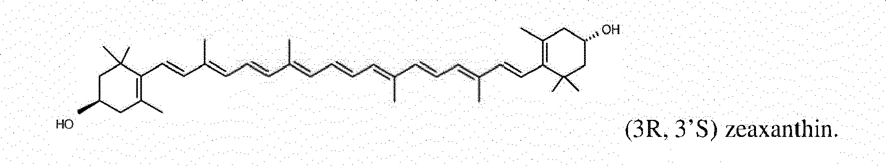

In a preferred embodiment, said composition comprises lutein and at least one zeaxanthin isomer, present in a lutein:zeaxanthin isomer ratio of about 5: 1. Preferably, the lutein and at least one zeaxanthin isomer are extracted from *Tagetes erecta,* and are free from pesticides, dioxin and genetically modified organism (GMO) or genetically engineered organism (GEO). More preferably, the lutein and at least one zeaxanthin isomer are in a crystalline form, such as, for example, lutein/zeaxanthin FloraGlo^{®}, commercially available from LCF.

In an embodiment, lutein is provided as lutein free 20%. In an embodiment, zeaxanthin isomer is provided as zeaxanthin 20%. Preferably, lutein and zeaxanthin isomer are provided as concentrated oleoresin from *Tagetes erecta* flowers, preferentially titrated respectively at 200 mg of lutein/g and 200 mg of zeaxanthin/g, respectively.

In a first embodiment of the invention, the amount of lutein in the composition ranges from 1 to 10 mg, preferably from 2.5 to 7.5 mg, more preferably is about 5 mg. Preferably, lutein ranges from 10-30% in weight to the weight of active agents in the composition.

In a second embodiment of the invention, the amount of lutein in the composition ranges from 1 to 25 mg, preferably from 3 to 15 mg, more preferably is about 5 mg. Preferably, lutein ranges from 3-5% in weight to the weight the composition.

In a first embodiment of the invention, the amount of at least one zeaxanthin isomer in the composition ranges from 0.1 to 2 mg, preferably from 0.5 to 1.5 mg, more preferably is about 1 mg. Preferably, zeaxanthin ranges from 1.5-8% in weight to the weight of active agents in the composition.

In a second embodiment of the invention, the amount of at least one zeaxanthin isomer in the composition ranges from 0.25 to 5 mg, preferably from 1 to 3 mg, more preferably is about 1 mg. Preferably, zeaxanthin ranges from 0.6-1% in weight to the weight the composition.

In an embodiment of the invention, lutein and zeaxanthin isomer are micro-encapsulated with a biopolymer, preferably made of prolamin. In another embodiment, lutein and zeaxanthin isomer are micro-encapsulated with a biopolymer made of PCADK.

In an embodiment of the invention, lutein, zeaxanthin isomer and the enzyme are micro-encapsulated in the same particles. According to an embodiment, lutein and zeaxanthin isomer are further coated with a polymer, to facilitate their digestive absorption. In an embodiment, said polymer is chitosan and/or hyaluronic acid. According to an embodiment, the lutein, at least one zeaxanthin isomer and the enzyme are present in the same chitosan and/or hyaluronic acid coated particles.

According to an embodiment, the composition of the invention further comprises one or more other non-enzymatic antioxidant agent, selected from the group comprising vitamins, such as, for example, Vitamin A, Vitamin C, Vitamin E or Vitamin B19; minerals such as, for example, copper, manganese, zinc or selenium; vegetal antioxidants such as, for example, anthocyanosids, glucosinolates, lycopene or polyphenols such as, for examples, flavonoids, catechins, stilbenoids, isoflavones; Coenzyme Q10 and poly-unsaturated lipid, such as, for example, omega3.

According to an embodiment of the invention, the composition of the invention does not comprise β-carotene.

According to an embodiment, the composition further comprises excipients, such as for example coloring agents, such as, for example, titane dioxide (E171), iron dioxide (E172) and brilliant black BN (E151); flavoring agents; thickeners, such as, for example, glycerol monostearate; sweeteners; coating agents, such as, for example, refined colza oil, soya oil, peanut oil, soya lecithin or fish gelatin; diluting agents, such as, for example, lactose, monohydrated lactose or starch; binding agents, such as, for example, povidone, pregelatinized starch, gums, saccharose, polyethylene glycol (PEG) 4000 or PEG 6000; disintegrating agents, such as, for example, microcrystalline cellulose or sodium carboxymethyl starch, such as, for example, sodium carboxymethyl starch type A; lubricant agents, such as, for example, magnesium stearate; flow agent, such as, for example, colloidal anhydrous silica, etc.... Advantageously, the above-mentioned excipients are present in an amount ranging from 50 to 80% in weight to the total weight of the composition.

According to an embodiment, the composition further comprises water. According to one embodiment, the amount of water in the composition of the invention ranges from about 2.5 to about 20 mg, preferably from about 5 to about 15 mg, more preferably is of about 10.8 mg. In one embodiment of the invention, the amount of water in the composition ranges from 0 to 10 % in weight to the total weight of the excipients, preferably from about 1 to about 5% w/w, more preferably is of about 3.3% w/w.

According to an embodiment, the composition of the invention is an intimate mixture of SOD with lutein and at least one zeaxanthin stereoisomer. In a further embodiment, the composition consists of SOD in association with lutein and at least one zeaxanthin stereoisomer and suitable excipients.

According to an embodiment, the composition of the invention is to be orally administered.

According to the invention, the composition may be in a solid form (pill, tablet, capsule, soft gelatin capsule, sugar-coated pill, orodispersing/orodisintegrating tablet, powder, effervescent tablet, etc...), in a liquid form (injectable solution, drinkable solution, buccal spray, eye drops, ointment...), or in the form of a gel.

The invention also relates to a kit of parts comprising the composition of the invention, and preferably comprising two parts. According to an embodiment, a first part of the kit comprises the enzyme, optionally protected by a polymer such as, for example prolamin, and a second part comprises lutein and at least one stereoisomer of zeaxanthin. According to an embodiment, both parts of the kit are orally administered. In an embodiment, each part of the kit is independently in a solid form (pill, tablet, capsule, soft gelatin capsule, sugar-coated pill, orodispersing/orodisintegrating tablet, powder, effervescent tablet, etc ... ), in a liquid form (injectable solution, drinkable solution, buccal spray, eye drops, ointment ...), or in the form of a gel.

According to an embodiment, one of the first and second parts of the kit is formulated for sustained release thereof. In an embodiment, when formulated for sustained release thereof, the part of the kit is in a solid form, preferably is a coated pill, a coated tablet, a soft gelatin tablet or a pill with modified release kinetic. When the part of the kit is not formulated for sustained release thereof and is in a solid form, it is preferably a non-coated pill, a non-coated tablet, a soluble pill, a dispersible pill or an orodispersible pill.

According to a first embodiment of the invention, the first and second parts of the kit of parts are administered simultaneously to a subject. According to a preferred embodiment, both parts of the kit are administered once a day, preferably in the morning.

According to a second embodiment of the invention, the first and second parts of the kit are administered sequentially to a subject. According to this embodiment, the first part comprising SOD is administered 12 hours before or after the second part comprising lutein and at least one zeaxanthin isomer. Surprisingly, the Applicant showed that the effect of the kit of part of the invention, specifically on SOD activity, photoreceptor viability or DHE intensity, is strongly enhanced when both parts of the kit are administered within an interval of 12-hours (see Examples). According to a preferred embodiment, the first and second parts of the kit are administered once a day, preferably the first one in the morning and the second one in the evening.

The composition or the kit may be used for maintaining visual health. The composition or the kit may be used for maintaining or improving visual comfort.

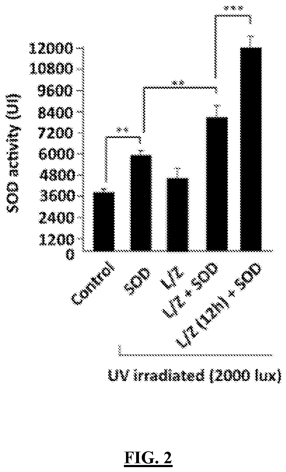

In one embodiment of the invention, the composition or the kit of the invention is for enhancing SOD activity in retinal pigment epithelial cells. The Applicant showed that the combination of SOD, of lutein and of at least one isomer of zeaxanthin resulted in an unexpected synergic effect, more preferably in a potentiating effect on SOD activity (Figure 2).

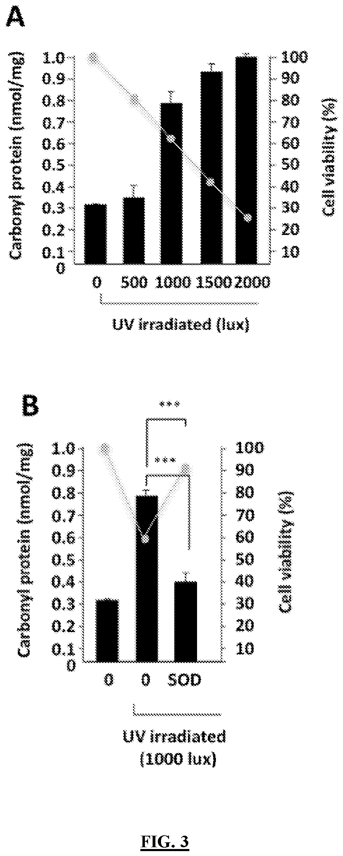

The composition or the kit can preserve cells from damages induced by oxidative stress, such as, for example, carbonylation of proteins (Figure 3).

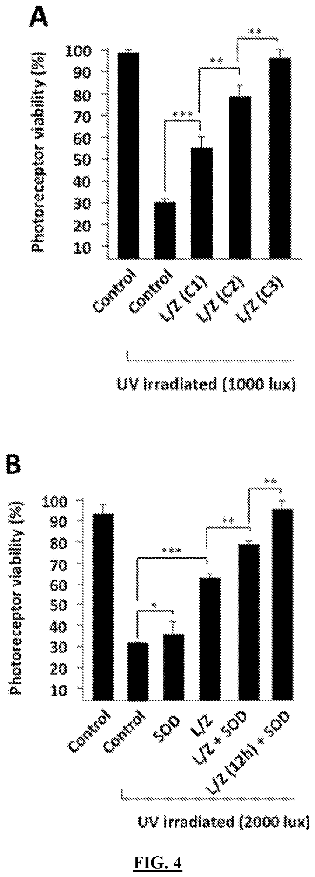

In one embodiment of the invention, the composition or the kit of the invention is for preserving photoreceptor cell viability. The Applicant showed that the combination of SOD, of lutein and of at least one isomer of zeaxanthin resulted in an unexpected synergic effect, more preferably in a potentiating effect on photoreceptor viability (Figure 4).

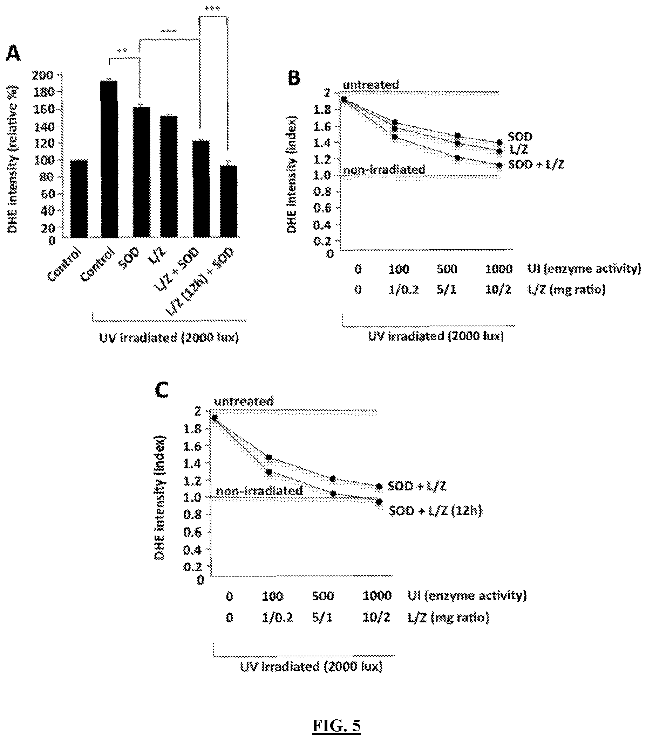

In one embodiment of the invention, the composition or the kit of the invention is for reducing the quantity of ROS in the retina (Figure 5).

In one embodiment of the invention, the composition or the kit of parts of the invention is in the form of a functional food, a nutraceutical composition, a food or dietary supplement, a pharmaceutical composition or a medicament.

The invention also relates to a use of a functional food, a nutraceutical composition or a food or dietary supplement, comprising the composition of the invention, or the kit of parts of the invention, as previously described. The functional food, the nutraceutical composition or the food or dietary supplement can maintain or improve visual comfort.

According to a third embodiment, the functional food, nutraceutical composition or food or dietary supplement is for use in preventing ocular aging.

According to an embodiment, the functional food, nutraceutical composition or food or dietary supplement is suitable for oral administration, and is preferably in a solid form, in a liquid form or in the form of a gel.

According to an embodiment, the functional food, nutraceutical composition or food or dietary supplement is to be ingested once a day, during a period of time ranging from 1 to 120 days, preferably from 10 to 50 days, more preferably during about 30 days.

In one embodiment of the invention, the composition is such that a daily amount of vegetal extract including SOD ranging from 10 to 500 mg, preferably from 50 to 200 mg, more preferably of about 100 mg is provided.

In one embodiment of the invention, the composition is such that a daily amount of lutein ranging from 1 to 10 mg, preferably from 2.5 to 7.5 mg, more preferably of about 5 mg is provided.

In one embodiment of the invention, the composition is such that a daily amount of at least one zeaxanthin isomer ranging from 0.1 to 2 mg, preferably from 0.5 to 1.5 mg, more preferably of about 1 mg is provided.

According to a first embodiment, the functional food, nutraceutical composition or food or dietary supplement is recommended to a subject with a genetic predisposition to or a risk of developing an ocular disease, disorder or condition related to oxidative stress, such as, for example, Age-related Macular Degeneration (AMD), diabetic retinopathies, cataract and retinitis pigmentosa.

According to a second embodiment, the functional food, nutraceutical composition or food or dietary supplement is recommended to a subject exposed to environmental stress, such as, for example, tobacco, air pollution, UV light and the like causing oxidative stress to the eye.

The invention also relates to a pharmaceutical composition comprising the composition of the invention, or the kit of parts of the invention, as previously described, in association with at least one pharmaceutically acceptable excipient as defined in claim 17. The invention also relates to a medicament comprising the composition of the invention, or the kit of parts of the invention, as previously described as defined in claims 19 and 20.

According to a first embodiment, the pharmaceutical composition or the medicament of the invention is orally administered. Advantageously, the medicament or the pharmaceutical composition is in a solid form, in a liquid form or in the form of a gel.

According to a second embodiment, the medicament or the pharmaceutical composition of the invention is topically administered, preferably is applied topically on the eye surface. Advantageously, the medicament or the pharmaceutical composition is an eye drop, an ointment and the like.

According to a third embodiment, the medicament or the pharmaceutical composition of the invention is intraocularly administered. By intraocularly is meant an injection directly in the eye ball, preferably inside the vitreous chamber.

According to a fourth embodiment, the medicament or the pharmaceutical composition of the invention is injected, preferably systemically injected.

Advantageously, when injected, the composition comprises recombinant SOD.

The pharmaceutical composition or the medicament used in the invention can maintain visual health.

According to one embodiment of the invention, the pharmaceutical composition or the medicament of the invention is useful for preventing, stabilizing or treating a retinal pathology. In an embodiment, said retinal pathology is associated with oxidative stress, such as, for example, Age-related Macular Degeneration (AMD), diabetic retinopathies, cataract and retinitis pigmentosa.

The composition used in the invention can prevent and/or treat ocular ageing.

The pharmaceutical composition used in the invention can treat pain, such as, for example, pain associated with an ocular disease, disorder or condition.

According to an embodiment, the composition is for use before or after surgery of cataract.

The composition used in the invention may be a visual care product, which means that it maintains visual health.

According to another embodiment, the pharmaceutical composition of the invention or the medicament of the invention is administered 1 to 5 times, preferably from 2 to 4 times a day, during a recommended period of treatment of at least 1 to 3 weeks. Such treatment may be repeated one to three times. In severe pathologies, the recommended period of treatment may be indefinite, which means that the treatment may be a chronic treatment.

In one embodiment of the invention, the composition is such that a daily amount of vegetal extract including SOD ranging from 100 to 5000 mg, preferably from 200 to 2000 mg, more preferably from 250 to 1000 mg is provided.

In one embodiment of the invention, the composition is such that a daily amount of lutein ranging from 2 to 100 mg, preferably from 5 to 50 mg, more preferably from 10 to 20 mg is provided.

In one embodiment of the invention, the composition is such that a daily amount of at least one zeaxanthin isomer ranging from 1 to 3, preferably from 1.5 to 2.5, more preferably of about 2 mg, is provided.

In a preferred embodiment, the composition or the kit of parts of the invention is administered to a subject in need thereof, through an oral way. According to an embodiment, the subject is an animal, preferably a mammal (cat, dog, hamster, rabbit, mouse, gerbil, rat, Guinea pig, human...), more preferably a human.

For maintaining or improving visual comfort and/or for preventing ocular aging in a subject in need thereof, the composition, the kit of part or the nutraceutical composition, food or dietary supplement or functional food may be administered to said subject in a nutraceutically effective amount. As used herein, the expression "nutraceutically effective amount" refers to the amount of a nutraceutical composition, food or dietary supplement or functional food necessary and sufficient for providing a physiological benefit or alleviating a discomfort.

The administration may be or comprise oral administration of the composition or kit of part.

For maintaining or improving visual comfort and/or for preventing ocular aging in a subject in need thereof, said subject may be administered with:
- a vegetal extract including SOD in an amount ranging from 10 to 500 mg, preferably from 50 to 200 mg, more preferably of about 100 mg,
- lutein in an amount ranging from 1 to 10 mg, preferably from 2.5 to 7.5 mg, more preferably of about 5 mg, and
- at least one zeaxanthin isomer in an amount ranging from 0.1 to 2 mg, preferably from 0.5 to 1.5 mg, more preferably of about 1 mg.

For maintaining visual health, for preventing, stabilizing and/or treating a retinal pathology in a subject in need thereof, a pharmaceutically effective amount of the composition, the kit of part, the pharmaceutical composition or the medicament used in the inventionaid may be administered to said subject. As used herein, the term "pharmaceutically effective amount" refers to the amount of a pharmaceutical composition or of a medicament necessary and sufficient for preventing the symptoms of a disease, slowing down or stopping the progression, aggravation, or deterioration of one or more symptoms of a disease, or condition; or alleviating the symptoms of a disease or condition; or curing the disease or condition.

In one embodiment, the retinal pathology is associated with oxidative stress. Examples of retinal pathologies associated with oxidative stress include, but are not limited to, AMD, diabetic retinopathies, cataract and retinitis pigmentosa.

In a first embodiment, the administration may be or comprise oral administration of the composition or kit of part used in the invention. In a second embodiment, administration may be or comprise topical administration of the composition or kit of part used in the invention. In a third embodiment, administration may be or comprise intraocular, preferably the intravitreal, administration of the composition or kit of part used in the invention. In a fourth embodiment, administration may be or comprise systemic administration of the composition or kit of part used in the invention.

For preventing, stabilizing or treating a retinal pathology, preferably a retinal pathology associated with oxidative stress, a composition or a kit of parts comprising SOD, preferably SOD micro-encapsulated with gliadin, lutein and at least one zeaxanthin stereoisomer may be administered to a subject in need thereof.

For maintaining visual health and for preventing, stabilizing and/or treating a retinal pathology in a subject in need thereof, said subject may be adminsistered with:
- a vegetal extract including SOD in an amount ranging from 50 to 2000 mg, preferably from 100 to 1000 mg, more preferably of about 250 mg,
- lutein in an amount ranging from1 to 25 mg, preferably from 3 to 15 mg, more preferably of about 5 mg, and
- at least one zeaxanthin isomer in an amount ranging from0.25 to 5 mg, preferably from 1 to 3 mg, more preferably is about 1 mg.

### BRIEF DESCRIPTION OF THE DRAWINGS

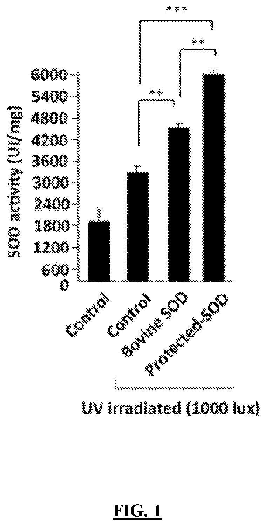

**Figure 1** is a histogram showing a comparison of Bovine and vegetal protected SOD on UV-irradiated RPE. SOD activity was measured in RPE stimulated by UV (1000 lux) treated with excipient (control), Bovine SOD origin (SIGMA^{®}) or protected-vegetal SOD origin compared to non-irradiated RPE. Values are means±SD. **p < 0.01, ***p < 0.001.
**Figure 2** is a histogram showing the plasmatic c-SOD (cellular-SOD) activity on UV-irradiated mice. Non-UV irradiated mice, n=10; UV-irradiated mice, n=12; UV-irradiated mice fed lutein/zeaxanthine (L/Z) diet, n=5; UV-irradiated mice fed SOD diet, n=10; UV-irradiated mice fed lutein/zeaxanthine + SOD diet, n=15; UV-irradiated mice fed lutein/zeaxanthine 12 hours before SOD diet, n=15. Values are means±SD. **p < 0.01, ***p < 0.001.
**Figure 3** is a combination of two histograms, showing the effect SOD on RPE cells irradiated with UV. A- UV-irradiation increased carbonyl protein quantity on RPE cells in a dose dependent of lux intensity (black histograms). In correlation with oxydative stress and carbonyl protein concentration, UV induced cell death (grey curve). **B-** SOD exposure reversed apoptosis induced by UV in RPE. Values are means±SD. **p < 0.01, ***p < 0.001.
**Figure 4** is a combination of two histograms, showing the effect of lutein and zeaxanthin on the viability of UV-irradiated photoreceptors. UV-irradiation increased photoreceptor apoptosis in cell culture (**A**) and *in vivo* (**B**) or in retina culture explants (i.e. two *in vivo* results, data not show). Lutein/zeaxanthin decreased photoreceptor apoptosis in a dose dependent manner (concentration; C1 to C3). Cell viability was assessed using DHE and AnnexinV/PI stainings. Values are means±SD. *p < 0.5, **p < 0.01, ***p < 0.001.
**Figure 5** is a combination of graphs, showing that UV-induced oxidative stress in the retina is reduced by the addition of both lutein/zeaxanthine and SOD. The fluorescence intensity in the inner nuclear layer relative to that of un-irradiated mice was measured by the Image J program. **A-** Non-UV irradiated mice, n=10; UV-irradiated mice, n=12; UV-irradiated mice fed lutein/zeaxanthine (L/Z) diet, n=5; UV-irradiated mice fed SOD diet, n=10; UV-irradiated mice fed lutein/zeaxanthine + SOD diet, n=15; UV-irradiated mice fed lutein/zeaxanthine 12 hours before SOD diet, n=15; in a lutein/zeaxanthin and SOD dose dependence (**B**) and efficiency increased with 12 hours pre-treatment with lutein/zeaxanthin (**C**). Values are means±SD. **p < 0.01, ***p < 0.001.

### EXAMPLES

The present invention is further illustrated by the following non-limiting examples.

### Example 1:

### Nutraceutical product (per unitary dosage)

| **Active agents** | **Weight (mg)** |
|---|---|
| SOD | 50-200, preferably 100 |
| Lutein | 2.5-7.5, preferably 5 |
| Zeaxanthin | 0.5-1.5, preferably 1 |
| Excipients | 53-350, preferably 150 |

### Example 2:

### Nutraceutical product (per unitary dosage - preferred unitary dosage form is a capsule)

| **Ingredients** | | **Quantity (mg)** | **Dosage per capsule (mg)** |
|---|---|---|---|
| Active agents | SOD (GliSODin^{®} from Isocell) | 50-200 | 100 |
| | Lutein free 20 % | 12.5-37.5 | 26.3 |
| | | *Equivalent 2.5-7.5 lutein* | *Equivalent 5.26 lutein* |
| | zeaxanthin free 20 % | 2.5-7.5 | 5.0 |
| | | *Equivalent 0.5-1.5 zeaxanthin* | *Equivalent 1 zeaxanthin* |
| | *Total of active agents* | *65-245* | *131.3* |
| Excipients | Refined colza oil | 200-250 | 230 |
| | Fish gelatin | 50-75 | 63.9 |
| | Glycerol monostearate (E471 - thickener) | 10-30 | 19.0 |
| | Water | 5-15 | 10.8 |
| | Colouring agents | 0.1-10 | 1.3 |
| | *Total of excipients* | *265.1-380* | *325* |

### Nutraceutical product (in percentage* per unitary dosage - preferred unitary dosage form is a capsule)

| **Ingredients** | | **Range of Percentage** | **Preferred percentage** |
|---|---|---|---|
| Active agents | SOD (GliSODin^{®} from Isocell) | 50-95 | 76.2 |
| | Lutein free 20 % | 10-30 | 20.0 |
| | zeaxanthin free 20 % | 1.5-8 | 3.8 |
| | *Total of active agents* | *100* | *100* |
| Excipients | Refined colza oil | 50-90 | 70.8 |
| | Fish gelatin | 10-30 | 19.7 |
| | Glycerol monostearate (E471 - thickener) | 0-10 | 5.8 |
| | Water | 0-10 | 3.3 |
| | Colouring agents | 0-5 | 0.4 |
| | *Total of excipients* | *100* | *100* |

| | | | |
|---|---|---|---|
| *: percentages are in weight to the total weight of active ingredients or excipients respectively. | | | |

### Example 3:

### Pharmaceutical composition (tablet)

| **Ingredients** | | **mg** | **Percentage^{∗}** |
|---|---|---|---|
| Active agents | SOD (GliSODin^{®} from Isocell) | 250.00 mg | 35.6 % |
| | Lutein free 20 % | 25.00 mg | 3.6 % |
| | Zeaxanthin 20 % | 5.00 mg | 0.7 % |
| Excipients | Monohydrated Lactose | 299.00 mg | 42.7 % |
| | Povidone | 14.00 mg | 2.0 % |
| | Microcrystalline Cellulose | 74.00 mg | 10.6 % |
| | Sodium carboxymethyl starch (type A) | 28.00 mg | 4.0 % |
| | Magnesium stearate | 5.50 mg | 0.8 % |
| | **TOTAL** | **700.5 mg** | **100.0 %** |

| | | | |
|---|---|---|---|
| *: percentages are in weight to the total weight of the tablet. | | | |

### Example 4:

### Pharmaceutical composition (capsule, size 0)

| **Ingredients** | | **mg** | **Percentage*** |
|---|---|---|---|
| Active agents | SOD (GliSODin^{®} from Isocell) | 250.00 mg | 33.5 % |
| | Lutein free 20 % | 25.00 mg | 3.3 % |
| | Zeaxanthin 20 % | 5.00 mg | 0.7 % |
| Excipients | Monohydrated lactose | 294.00 mg | 39.4 % |
| | Pregelatinized starch | 158.5 mg | 21.3 % |
| | Colloidal anhydrous silica | 6.6 mg | 0.9 % |
| | Magnesium stearate | 6.6 mg | 0.9 % |
| | **TOTAL** | **745.7 mg** | **100.00 %** |

| | | | |
|---|---|---|---|
| ^{∗}: percentages are in weight to the total weight of the capsule. | | | |

### Example 5:

### Pharmaceutical composition (soft gelatin capsule)

| **Ingredients** | | **mg** | **Percentage^{∗}** |
|---|---|---|---|
| Active agents | SOD (GliSODin^{®} from Isocell) | 250.00 mg | 43.1 % |
| | Lutein free 20 % | 25.00 mg | 4.3 % |
| | Zeaxanthin 20 % | 5.00 mg | 0.9 % |
| Excipients | Peanut oil | 298.00 mg | 51.4 % |
| | Soya lecithin | 2.00 mg | 0.3 % |
| | **TOTAL** | **580.00 mg** | **100.00 %** |

| | | | |
|---|---|---|---|
| *: percentages are in weight to the total weight of the soft gelatin capsule. | | | |

### Example 6:

### Materials and Methods

### Material

When not specified, SOD is a vegetal extract containing SOD associated to gliadin to protect it and contribute to its intestinal absorption. 1 mg of SOD/gliadin mix has a SOD activity of at least 1.1 UI/mg.

### Methods

- Animals: C57BL/6 mice were UV exposed (2000 lux during 5 hours). Mice fed a lutein/zeaxanthin (1 to 10 mg range), SOD (100 to 1000 unit range) or a mix of both-supplemented.
- Retina explants: The eyes were enucleated and placed in a dish containing Nutrient medium R16 with the addition of 10% fetal calf serum (Gibco), 2.56 mg/L cytidine 5-diphosphocholine, 1.28 mg/L cytidine diphosphoethanolamine, used as culture medium. An eyeball incision was made behind the limbus and the cornea-iris complex, lens and vitreous were removed. The explants were placed lying flat on a 10 mm nitrocellulose membrane (Millipore, pore size 0.45 pm) with the vitreal side facing upwards. The culture dishes were placed in an incubator with 5% C02 in air, 100% humidity at 37° Celsius.
- Cell cultures: Briefly, human cells from retina (Retinal pigment epithelial - RPE cells) ARPE-19 type (ATTC Ref. CRL-2302) were used for culture experiments. RPE cells were seeded at 10⁶ cells/mL and plated at 37°C, 5% CO2, using Dulbecco's medium (Invitrogen^{®}) supplemented with 10% SVF, 1.2 g/L sodium bicarbonate, 2.5 mM L-glutamine, 15 mM HEPES and 0.5 mM sodium pyruvate. Mice retinas were dissected and dissociated by trypsin digestion followed by mechanical dissociation. After dissociation, the cells were cultured in photoreceptor-defined medium. Approximately 10⁶ cells were seeded on 35-mm diameter dishes. Cultures were incubated at 36°C in a humidified atmosphere of 5% CO2.
- SOD activity measurement *in vitro:* SOD activity is measured using RANSOD KIT (RANDOX^{®}) in ARPE-19 (Retinal pigment epithelial - RPE cells) (ATTC Ref. CRL-2302) under treatment conditions. Cells are cultured at 10⁶ cells/ml in 6 well plates (NUNC^{®}) at 37°C, 5% CO₂, using Dulbecco's medium (INVITROGEN^{®}) supplemented with 10% SVF, 1.2 g/L sodium bicarbonate, 2.5 mM L-glutamine, 15 mM HEPES, 0.5 mM sodium pyruvate. After treatment, cells were harvested using trypsine and lysed by sonication. Protein extracts were quantified, and SOD activity was measured following RANDOD KIT instruction. Results are expressed in SOD activity (UI)/mg of protein ratio.
- SOD activity measurement *in vivo:* Briefly, blood samples (0.5 ml) were collected from mice exposed to UV irradiation treated or not with SOD, lutein/zeaxanthin (L/Z), SOD + L/Z or with time delay (12 hours) of L/Z and SOD administration. Blood samples were centrifuged at 1000g, 30 minutes at 4°C, erythrocyte pellets were reconstituted and washed using 0.5 ml of 0,172 mol/L (pH=7.6) Tris HCl buffer. Erythrocytes were lysed using 3 ml of 0.011 ml/L (pH=7.6) Tris HCl buffer, and membranes were separated using 2000 g, 40 minutes centrifugation procedures. Membrane pellets were washed using 0.011 ml/L (pH=7.6) Tris HCl buffer (3 times) and SOD activity was measured using RANSOD KIT (RANDOX^{®}) as previously described.
- Biotin-dUTP Nick-end Labeling (TUNEL): Apoptosis was examined by TUNEL assay (DeadEnd Fluorometric TUNEL system, Promega, Madison, WI). Sections were counterstained with 4,6-diamidino-2-phenylindole (DAPI) after the reaction. Only sections with optic nerve stumps were used and images were captured from the central, the middle, and from the peripheral retina. The number of TUNEL-positive nuclei in both ganglion cell layer and inner nuclear layer were counted.
- ROS measurement in retina: Eyes were enucleated and immediately frozen in OCT compound (Sakura Finetek, Torrance, CA, USA). Retina explants were also fixed in OCT. Unfixed cryosections (10 µm) using a microtome (Microm HM 315R, Heidelberg, Germany), were incubated with dihydroethidium (DHE; Invitrogen-Molecular Probes, Eugene, OR, USA) (5 µmol/L) for 20 min at 37°C. DHE specifically reacts with intracellular superoxide anion and is converted to the red fluorescent compound ethidium in nuclei. The sections were examined using a microscope possessing a digital camera (Carl Zeiss, Jena, Germany), and the intensity of the staining was measured in the posterior retina, using the Image J software (National Institutes of Health, Bethesda, MD, USA).
- Apoptosis assay: For TUNEL staining, the cells were fixed with 2% PFA for 15 minutes and stored in 70% ethanol for 48 hours at -20°C. Before labeling, cells were washed twice with PBS for 5 minutes at room temperature. Samples were incubated with TdT reaction mixture (0.05 mM BrdUTP, 0.3 U/µL TdT) at 37°C for 1 hour. The reaction was stopped using buffer (300 mM NaCl, 30 mM sodium citrate; pH=7.4) at room temperature. Negative controls were prepared by omitting TdT. BrdU was detected using an anti-BrdU monoclonal antibody and Annexin V staining was performed. The cells were washed twice in cold PBS, labeled with Annexin V/PI buffer and were then analyzed by fluorescence.

### Results

*Vegetal protected SOD is more efficient than Bovine SOD to stimulate SOD activity in UV-irradiated RPE cells.* The treatment of UV-irradiated RPE cells by SOD enhances measured SOD activity (in UI/mg). Moreover, vegetal SOD protected with gliadin is more efficient than naked bovine SOD (Figure 1).

*Effects of SOD and*/*or lutein*/*zeaxanthin on c-SOD activity:* As previously shown *in vitro* (see above and figure 1), SOD also enhances measured c-SOD activity *in vivo* in irradiated mice. In parallel, the treatment of UV-irradiated mice by lutein/zeaxanthin slightly stimulates c-SOD activity. SOD and lutein/zeaxanthin exhibit an unexpected synergic effect, which is a potentiating effect on the stimulation of c-SOD activity *in vivo.* Surprisingly, the potentiating effect is enhanced when both products are administered within an interval of 12 hours (figure 2).

*Protection of cells from oxidative damages by SOD and*/*or lutein*/*zeaxanthin:*
UV-irradiation of RPE cells leads to oxidative damages, such as protein carbonylation and to a decrease in cell viability. The treatment of cultured RPE cells with SOD preserves those cells from such damages (figure 3).
SOD and lutein/zeaxanthin (dose dependent effect) preserve photoreceptor viability after UV-irradiation, both *in vivo* and *ex vivo.* Both products show a synergic effect, more specifically a potentiating effect, which is enhanced when they are administered 12 hours apart one from another (figure 4).
SOD and lutein/zeaxanthin preserve *in vivo* the retina from oxidative stress (measured by DHE intensity). Both products still show a synergic effect, which is enhanced when they are administered 12 hours apart one from another (figure 5).

## Claims

1. A composition comprising the enzyme superoxide dismutase in association with lutein and at least one stereoisomer of zeaxanthin for use in a method of treating, preventing or stabilizing a retinal pathology.

2. A composition comprising the enzyme superoxide dismutase in association with lutein and at least one stereoisomer of zeaxanthin for use in a method of treating, preventing or stabilizing Age-related Macular Degeneration (AMD), diabetic retinopathies, cataract or retinitis pigmentosa.

3. The composition for use according to of claim 1 or 2, wherein the enzyme superoxide dismutase (SOD) is of natural origin, and the composition comprises a vegetal extract including SOD, preferably an extract of *Cucumis melo* LC, preferably the amount of vegetal extract including superoxide dismutase ranges from 10 to 2000 mg, preferably from 50 to 1000 mg, more preferably 100-500 mg.

4. The composition for use according to any one of claims 1 to 3, wherein enzymatic activity of said enzyme is of at least 1.1 UI/mg.

5. The composition for use according to anyone of claims 1 to 4, wherein the enzyme is micro-encapsulated with a biopolymer, preferably a prolamine or PCADK.

6. The composition for use according to claim 5, wherein the micro-encapsulated enzyme is further coated with a polymer, preferably chitosan or hyaluronic acid.

7. The composition for use according to anyone of claims 1 to 6, wherein the at least one zeaxanthin stereoisomer is selected from the group consisting of (3R, 3'R) zeaxanthin, (3S, 3'S) zeaxanthin, and (3R, 3'S) zeaxanthine.

8. The composition for use according to anyone of claims 1 to 7, wherein the amount of lutein ranges from 0.5 to 25 mg, and the amount of at least one zeaxanthin stereoisomer ranges from 0.1-5 mg.

9. The composition for use according to anyone of claims 1 to 8, wherein the lutein and at least one zeaxanthin stereoisomer are present in the composition in a ratio lutein:zeaxanthin isomer of about 5:1.

10. The composition for use according to anyone of claims 1 to 9, wherein the association is an intimate mixture of the enzyme superoxide dismutase, lutein and at least one zeaxanthin stereoisomer.

11. A kit of parts comprising a composition as defined in anyone of claims 1 to 10, wherein the kit comprises a first part comprising the enzyme superoxide dismutase and a second part comprising lutein and at least one zeaxanthin stereoisomer.

12. The kit according to claim 11, wherein one of the first and second part is formulated for sustained release thereof.

13. The kit according to claim 11 or claim 12, wherein the first and second parts are to be administered to an individual simultaneously or sequentially, and when administered sequentially, the first part comprising the enzyme is to be administered 12 hours before or after the second part comprising lutein and at least one zeaxanthin stereoisomer.

14. Use of a functional food, nutraceutical composition, or dietary supplement comprising the composition as defined in anyone of claims 1 to 10 or comprising the kit of anyone of claims 11 to 13, for
- alleviating or preventing in a subject eye fatigue, eye itching, or eye winking, or;
- preventing and/or treating ocular aging

15. The use according to claim 14, wherein the functional food, nutraceutical composition, or dietary supplement comprises 50-200 mg, preferably about 100 mg enzyme, 2.5-7.5 mg, preferably about 5 mg lutein, and 0.5-1.5 mg, preferably about 1 mg zeaxanthin stereoisomer.

16. The use according to claim 14 or 15, wherein the functional food, nutraceutical composition, or dietary supplement is in a form suitable for being orally administered.

17. A pharmaceutical composition comprising a composition according to anyone of claims 1 to 10 in association with at least one pharmaceutically acceptable excipient for use in a method as defined in claim 1, 2, or 24.

18. A pharmaceutical composition comprising a kit of parts according to anyone of claims 11 to 13 in association with at least one pharmaceutically acceptable excipient.

19. A medicament comprising a kit of parts according to anyone of claims 11 to 13.

20. A medicament comprising a composition as defined in anyone of claims 1 to 10 for use in a method as defined in claim 1, 2, or 24.

21. The pharmaceutical composition for the use according to claim 17, the pharmaceutical composition or medicament according to claim 18 or 19, or the medicament for the use according to claim 20, comprising 100-1000 mg superoxide dismutase, 1-25 mg lutein and 0.5-5 mg zeaxanthin.

22. The pharmaceutical composition for the use according to claim 17 or 21, the pharmaceutical composition or medicament according to claim 18, 19 or 21, or the medicament for the use according to claim 20 or 21, in a form suitable for being administered orally, topically, intraocularly or systemically.

23. The composition for the use according to any one of claims 1 to 10, wherein said composition is a pharmaceutical composition or a medicament for the use in a subject in need thereof and
wherein said subject is an animal, preferably a mammal, such as, for example, cat, dog, hamster, rabbit, mouse, gerbil, rat, Guinea pig, human, more preferably said subject is a human.

24. A composition comprising the enzyme superoxide dismutase in association with lutein and at least one stereoisomer of zeaxanthin, or the kit of claim 11, for use in
- preserving photoreceptor cell viability; or
- reducing the quantity of reactive oxygen species (ROS) in the retina.

## Patentansprüche

1. Zusammensetzung, umfassend das Enzym Superoxid-Dismutase in Verbindung mit Lutein und mindestens einem Stereoisomer von Zeaxanthin, zur Verwendung in einem Verfahren zur Behandlung, Vorbeugung oder Stabilisierung einer Netzhautpathologie.

2. Zusammensetzung, umfassend das Enzym Superoxid-Dismutase in Verbindung mit Lutein und mindestens einem Stereoisomer von Zeaxanthin, zur Verwendung in einem Verfahren zur Behandlung, Vorbeugung oder Stabilisierung altersbedingter Makuladegeneration (AMD), diabetischen Retinopathien, Katarakt oder Retinitis pigmentosa.

3. Zusammensetzung zur Verwendung gemäß Anspruch 1 oder 2, wobei das Enzym Superoxid-Dismutase (SOD) natürlichen Ursprungs ist und die Zusammensetzung einen pflanzlichen Extrakt enthaltend SOD umfasst, vorzugsweise einen Extrakt von *Cucumis melo* LC, wobei vorzugsweise die Menge des pflanzlichen Extrakts, der Superoxid-Dismutase enthält, zwischen 10 bis 2000 mg liegt, vorzugsweise zwischen 50 bis 1000 mg, bevorzugter 100 bis 500 mg.

4. Zusammensetzung zur Verwendung gemäß einem der Ansprüche 1 bis 3, wobei die enzymatische Aktivität des Enzyms mindestens 1,1 UI/mg beträgt.

5. Zusammensetzung zur Verwendung gemäß einem der Ansprüche 1 bis 4, wobei das Enzym mit einem Biopolymer mikroverkapselt ist, vorzugsweise mit einem Prolamin oder PCADK.

6. Zusammensetzung zur Verwendung gemäß Anspruch 5, wobei das mikroverkapselte Enzym ferner mit einem Polymer beschichtet ist, vorzugsweise Chitosan oder Hyaluronsäure.

7. Zusammensetzung zur Verwendung gemäß einem der Ansprüche 1 bis 6, wobei das mindestens eine Stereoisomer von Zeaxanthin ausgewählt ist aus der Gruppe bestehend aus (3R, 3'R)-Zeaxanthin, (3S, 3'S)-Zeaxanthin und (3R, 3'S)-Zeaxanthin.

8. Zusammensetzung zur Verwendung gemäß einem der Ansprüche 1 bis 7, wobei die Menge an Lutein zwischen 0,5 bis 25 mg liegt und die Menge des mindestens einen Stereoisomers von Zeaxanthin zwischen 0,1 bis 5 mg liegt.

9. Zusammensetzung zur Verwendung gemäß einem der Ansprüche 1 bis 8, wobei das Lutein und mindestens eine Stereoisomer von Zeaxanthin in der Zusammensetzung in einem Verhältnis von Lutein:Zeaxanthin-Isomer von ungefähr 5:1 vorhanden sind.

10. Zusammensetzung zur Verwendung gemäß einem der Ansprüche 1 bis 9, wobei die Assoziation eine innige Mischung des Enzyms Superoxid-Dismutase, des Luteins und des mindestens einen Stereoisomers von Zeaxanthin ist.

11. Kit umfassend eine Zusammensetzung wie in einem der Ansprüche 1 bis 10 definiert, wobei der Kit einen ersten Teil umfasst umfassend das Enzym Superoxid-Dismutase, und einen zweiten Teil umfassend Lutein und mindestens ein Stereoisomer von Zeaxanthin.

12. Kit gemäß Anspruch 11, wobei entweder der erste oder der zweite Teil für eine verzögerte Freisetzung davon formuliert ist.

13. Kit gemäß Anspruch 11 oder 12, wobei der erste und der zweite Teil einem Individuum gleichzeitig oder sequentiell verabreicht werden müssen, und wenn sie sequentiell verabreicht werden, der erste Teil umfassend das Enzym 12 Stunden vor oder nach dem zweiten Teil umfassend Lutein und mindestens ein Stereoisomer von Zeaxanthin verabreicht werden muss.

14. Verwendung eines funktionellen Lebensmittels, einer nutrazeutischen Zusammensetzung oder eines Nahrungsergänzungsmittels, umfassend die Zusammensetzung wie in einem der Ansprüche 1 bis 10 definiert, oder umfassend den Kit nach einem der Ansprüche 11 bis 13, zur
- Linderung oder Vorbeugung von Augenermüdung, Augenjucken oder Augenzwinkern in einem Subjekt oder
- zur Verbeugung und/oder Behandlung von Augenalterung.

15. Verwendung gemäß Anspruch 14, wobei das funktionelle Lebensmittel, die nutrazeutische Zusammensetzung oder das Nahrungsergänzungsmittel 50 bis 200 mg, vorzugsweise ungefähr 100 mg Enzym, 2,5 bis 7,5 mg, vorzugsweise ungefähr 5 mg Lutein und 0,5 bis 1,5 mg, vorzugsweise ungefähr 1 mg des Stereoisomers von Zeaxanthin umfasst.

16. Verwendung gemäß Anspruch 14 oder 15, wobei das funktionelle Lebensmittel, die nutrazeutische Zusammensetzung oder das Nahrungsergänzungsmittel in einer zur oralen Verabreichung geeigneten Form vorliegt.

17. Pharmazeutische Zusammensetzung umfassend die Zusammensetzung gemäß einem der Ansprüche 1 bis 10 in Verbindung mit mindestens einem pharmazeutisch verträglichen Hilfsstoff zur Verwendung in einem Verfahren wie in einem der Ansprüche 1, 2 oder 24 definiert.

18. Pharmazeutische Zusammensetzung umfassend den Kit gemäß einem der Ansprüche 11 bis 13 in Verbindung mit mindestens einem pharmazeutisch verträglichen Hilfsstoff.

19. Arzneimittel umfassend den Kit gemäß einem der Ansprüche 11 bis 13.

20. Arzneimittel umfassend die Zusammensetzung wie einem der Ansprüche 1 bis 10 definiert zur Verwendung in einem Verfahren wie in einem der Ansprüche 1, 2 oder 24 definiert.

21. Pharmazeutische Zusammensetzung zur Verwendung gemäß Anspruch 17, die pharmazeutische Zusammensetzung oder das Arzneimittel gemäß Anspruch 18 oder 19 oder das Arzneimittel zur Verwendung nach Anspruch 20, umfassend 100 bis 1000 mg Superoxid-Dismutase, 1 bis 25 mg Lutein und 0,5 bis 5 mg Zeaxanthin.

22. Pharmazeutische Zusammensetzung zur Verwendung gemäß Anspruch 17 oder 21, die pharmazeutische Zusammensetzung oder das Arzneimittel gemäß Anspruch 18, 19 oder 21 oder das Arzneimittel zur Verwendung gemäß Anspruch 20 oder 21 in einer Form, die geeignet ist, um oral, topisch, intraokular oder systemisch verabreicht zu werden.

23. Zusammensetzung zur Verwendung gemäß einem der Ansprüche 1 bis 10, wobei die Zusammensetzung eine pharmazeutische Zusammensetzung oder ein Arzneimittel zur Verwendung bei einem Subjekt ist, das dies benötigt, und wobei das Subjekt ein Tier ist, vorzugsweise ein Säugetier, wie zum Beispiel eine Katze, ein Hund, ein Hamster, ein Kaninchen, eine Maus, eine Wüstenrennmaus, eine Ratte, ein Meerschweinchen, ein Mensch, wobei das Subjekt vorzugsweise ein Mensch ist.

24. Zusammensetzung umfassend das Enzym Superoxid-Dismutase in Verbindung mit Lutein und mindestens einem Stereoisomer von Zeaxanthin, oder der Kit gemäß Anspruch 11, zur Verwendung bei der Erhaltung der Lebensfähigkeit der Photorezeptorzellen oder bei der Verringerung der Menge an reaktiven Sauerstoffspezies (ROS) in der Retina.

## Revendications

1. Composition comprenant l'enzyme superoxyde dismutase en association avec de la lutéine et au moins un stéréoisomère de zéaxanthine, pour une utilisation dans une méthode de traitement, prévention ou stabilisation d'une pathologie rétinienne.

2. Composition comprenant l'enzyme superoxyde dismutase en association avec de la lutéine et au moins un stéréoisomère de zéaxanthine, pour une utilisation dans une méthode de traitement, prévention ou stabilisation d'une dégénérescence maculaire liée à l'âge (AMD), de rétinopathie diabétiques, d'une cataracte ou d'une rétinite pigmentaire.

3. Composition pour une utilisation selon la revendication 1 ou 2, dans laquelle l'enzyme superoxyde dismutase (SOD) est d'origine naturelle, et la composition comprend un extrait végétal contenant de la SOD, de préférence un extrait de *Cucumis melo* LC, de préférence la quantité d'extrait végétal contenant de la superoxyde dismutase est dans la plage de 10 à 2000 mg, de préférence de 50 à 1000 mg, plus préférablement de 100 à 500 mg.

4. Composition pour une utilisation selon l'une quelconque des revendications 1 à 3, dans laquelle l'activité enzymatique de ladite enzyme est d'au moins 1,1 UI/mg.

5. Composition pour une utilisation selon l'une quelconque des revendications 1 à 4, dans laquelle l'enzyme est microencapsulée avec un biopolymère, de préférence une prolamine ou le PCADK.

6. Composition pour une utilisation selon la revendication 5, dans laquelle l'enzyme microencapsulée est en outre enrobée par un polymère, de préférence le chitosane ou l'acide hyaluronique.

7. Composition pour une utilisation selon l'une quelconque des revendications 1 à 6, dans laquelle l'au moins un stéréoisomère de zéaxanthine est choisi dans le groupe constitué par la (3R,3'R) zéaxanthine, la (35,3'S) zéaxanthine, et la (3R,3'S) zéaxanthine.

8. Composition pour une utilisation selon l'une quelconque des revendications 1 à 7, dans laquelle la quantité de lutéine est dans la plage de 0,5 à 25 mg, et la quantité de l'au moins un stéréoisomère de zéaxanthine est dans la plage de 0,1 à 5 mg.

9. Composition pour une utilisation selon l'une quelconque des revendications 1 à 8, dans laquelle la lutéine et au moins un stéréoisomère de zéaxanthine sont présents dans la composition en un rapport lutéine/isomère de zéaxanthine d'environ 5/1.

10. Composition pour une utilisation selon l'une quelconque des revendications 1 à 9, dans laquelle l'association est un mélange intime de l'enzyme superoxyde dismutase, de lutéine et d'au moins un stéréoisomère de zéaxanthine.

11. Kit de substances comprenant une composition telle que définie dans l'une quelconque des revendications 1 à 10, dans lequel le kit comprend une première partie comprenant l'enzyme superoxyde dismutase et une deuxième partie comprenant de la lutéine et au moins un stéréoisomère de zéaxanthine.

12. Kit selon la revendication 11, dans lequel l'une des première et deuxième parties est formulée pour une libération prolongée de celle-ci.

13. Kit selon la revendication 11 ou la revendication 12, dans lequel les première et deuxième parties sont destinées à être administrées à un individu simultanément ou successivement, et quand elles sont administrées successivement, la première partie comprenant l'enzyme est destinée à être administrée 12 heures avant ou après la deuxième partie comprenant de la lutéine et au moins un stéréoisomère de zéaxanthine.

14. Utilisation d'un aliment fonctionnel, d'une composition nutraceutique, ou d'un complément alimentaire comprenant la composition telle que définie dans l'une quelconque des revendications 1 à 10 ou comprenant le kit de l'une quelconque des revendications 11 à 13, pour
- soulager ou prévenir une fatigue oculaire, un prurit oculaire, ou un clignement des yeux chez un sujet; ou
- prévenir et/ou traiter un vieillissement oculaire.

15. Utilisation selon la revendication 14, dans laquelle l'aliment fonctionnel, la composition nutraceutique ou le complément alimentaire comprend 50 à 200 mg, de préférence environ 100 mg d'enzyme, 2,5 à 7,5 mg, de préférence environ 5 mg de lutéine, et 0,5 à 1,5 mg, de préférence environ 1 mg de stéréoisomère de zéaxanthine.

16. Utilisation selon la revendication 14 ou 15, dans laquelle l'aliment fonctionnel, la composition nutraceutique ou le complément alimentaire est sous une forme adaptée à une administration par voie orale.

17. Composition pharmaceutique comprenant une composition selon l'une quelconque des revendications 1 à 10 en association avec au moins un excipient pharmaceutiquement acceptable pour une utilisation dans une méthode telle que définie dans la revendication 1, 2 ou 24.

18. Composition pharmaceutique comprenant un kit de substances selon l'une quelconque des revendications 11 à 13 en association avec au moins un excipient pharmaceutiquement acceptable.

19. Médicament comprenant un kit de substances selon l'une quelconque des revendications 11 à 13.

20. Médicament comprenant une composition telle que définie dans l'une quelconque des revendications 1 à 10 pour une utilisation dans une méthode telle que définie dans la revendication 1, 2 ou 24.

21. Composition pharmaceutique pour une utilisation selon la revendication 17, composition pharmaceutique ou médicament selon la revendication 18 ou 19, ou médicament pour une utilisation selon la revendication 20, comprenant 100 à 1000 mg de superoxyde dismutase, 1 à 25 mg de lutéine et 0,5 à 5 mg de zéaxanthine.

22. Composition pharmaceutique pour une utilisation selon la revendication 17 ou 21, composition pharmaceutique ou médicament selon la revendication 18, 19 ou 21, ou médicament pour une utilisation selon la revendication 20 ou 21, sous une forme adaptée à une administration par voie orale, topique, intraoculaire ou systémique.

23. Composition pour une utilisation selon l'une quelconque des revendications 1 à 10, dans laquelle ladite composition est une composition pharmaceutique ou un médicament pour une utilisation chez un sujet en ayant besoin, et dans laquelle ledit sujet est un animal, de préférence un mammifère, comme par exemple un chat, un chien, un hamster, un lapin, une souris, une gerbille, un rat, un cochon d'Inde, un humain, mieux encore ledit sujet est un humain.

24. Composition comprenant l'enzyme superoxyde dismutase en association avec de la lutéine et au moins un stéréoisomère de zéaxanthine, ou kit selon la revendication 11, pour une utilisation dans
- la préservation de la viabilité de cellules photoréceptrices ; ou
- la réduction de la quantité d'espèces oxygénées réactives (ROS) dans la rétine.
